Europäisches Patentamt

European Patent Office

Office européen des brevets

(11) Publication number: 0 236 871
A2

## (12) EUROPEAN PATENT APPLICATION

(21) Application number: 87102769.4

(22) Date of filing: 26.02.87

(51) Int. Cl.⁴: B65D 47/20 , B65D 47/42

(30) Priority: 10.03.86 US 837535

(43) Date of publication of application:
16.09.87 Bulletin 87/38

(84) Designated Contracting States:
AT BE CH DE ES FR GB IT LI NL SE

(71) Applicant: Pharma-Gummi Wimmer West
GmbH
Stolberger Strasse 21-41
D-5180 Eschweiler(DE)

(72) Inventor: Waters, William E.
R.D. 4 Box 55
Line Road Malvern, PA 19355(US)
Inventor: Harman, Arlington R.
27 Spring Valley Road
Frazer, PA 19355(US)
Inventor: Friedrich, Peter
44 Riverside Avenue
Lyndhurst, NJ 07071(US)
Inventor: Walters, Ralph G.
89 Cepp Road
Perkiomerville, PA 18074(US)
Inventor: Voytilla, Joseph M.
1074 Grandview Circle North
Pottstown, PA 19464(US)

(74) Representative: Schmitt, Hans, Dipl.-Ing. et al
Patentanwälte Dipl.-Ing H. Schmitt Dipl.-Ing.
W. Maucher Dreikönigstrasse 13
D-7800 Freiburg(DE)

(54) Device for measuring and dispensing fluids.

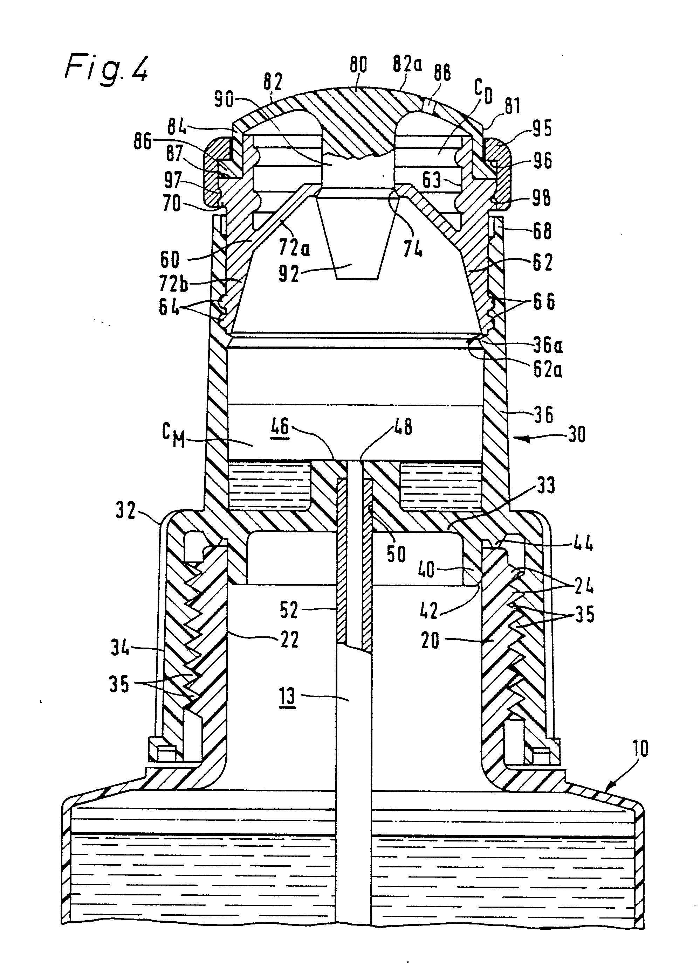

(57) Eine Vorrichtung (30) zum Dosieren und Spenden von Flüssigkeiten ist auf einen kompressiblen Behälter (10) aufschraubbar und weist eine Meßkammer (CM) sowie eine darüber angeordnete Spendekammer (CD) auf. Die Spendekammer wird von einem vorzugsweise aus Gummi bestehenden Befeuchtungsdeckel (80, 124) abgeschlossen. Die abzumessende Flüssigkeitsmenge ist abhängig von der Höhe mindestens eines in der Meßkammer (CM) angeordneten Meßvorsprunges (46, 102), durch den jede das vorbestimmte Maß überschreitende Flüssigkeit in den Behälter zurückfließt. Die auf diese Weise abgemessene Flüssigkeitsmenge kann nach Umdrehung des Behälters über ein durch den Befeuchtungsdeckel (80, 124) betätigtes Ventil (60, 125) in die Spendekammer gelangen und über Löcher des Befeuchtungsdeckels (80, 124) abgegeben werden (vergl. Fig. 4).

Fig. 4

## Vorrichtung zum Dosieren und Spenden von Flüssigkeiten

Die Erfindung betrifft eine Vorrichtung zum Dosieren und Spenden von Flüssigkeiten, z. B. von kosmetischen Emulsionen oder medizinischen Lösungen auf die Haut des Anwenders, mit einem unteren Kappenbereich und Befestigungselementen zum lösbaren Befestigen auf der Entnahmeöffnung eines Behälters, wobei in einem oberen Kappenbereich zum Abmessen einer vorgesehenen Flüssigkeitsmenge eine Meßkammer sowie eine Spendekammer mit einem durchlässigen Befeuchtungsdeckel angeordnet ist.

Solche Meß-und Spendebehälter sind bekannt. So zeigt z. B. das US-Patent Nr. 4 077 547 eine Behälteranordnung mit einem kompressiblen Behälter und einem Gewindehals, der einen Meß- und Spendebereich aufweist, welcher über dem Behälter angebracht ist, sowie eine Röhrenanordnung, die vom Meß-und Spendebereich in den Behälter reicht. Der Spendebereich weist hierbei ein senkrechtes röhrenförmiges Teil mit einer Bohrung auf, welche in Flüssigkeitsverbindung mit einer Auslaßöffnung steht, die am oberen abschließenden Ende dieses aufrechtstehenden rohrenförmigen Teiles angebracht ist. Die Röhrenanordnung reicht heirbei bis in die Bohrung des aufrechtstehenden Spendeteiles. Falls gewünscht, kann der Benutzer, um eine abgemessene Flüssigkeitsmenge aus dem Behälter auszuteilen, einfach auf die Wände des Behälters drücken, wodurch die Flüssigkeit durch die Röhre über die Bohrung des aufrechtstehenden Bereiches aus der Auslaßöffnung in die Meßkammer befördert wird. Falls das Volumen der aus dem Behälter in die Meßkammer geförderten Flüssigkeit dasjenige Volumen überschreitet, welches durch den oberen Rand des senkrechten rohrenförmigen Teiles bestimmt ist, fließt die überflüssige Flüssigkeit, sobald der Kompressionsdruck nachläßt, wieder zurück in den Behälter.

Ähnliche Meß-und Spendeanordnungen werden in den US-Patenten Nr. 3 347 420 und Nr. 3 581 953 gezeigt. Das an dieser Stelle auch zu erwähnende US-Patent Nr. 4 133 614 zeigt eine Deckelanordnung mit einer Vielzahl von Elementen, einschließlich eines Ventilteiles, eines elastischen porösen Elementes, eines flexiblen porösen Apparateunterbaues sowie eines Halteringes, um die Einzelteile zusammenzuhalten.

Obwohl die oben beschriebenen vorbekannten Anordnungen generell für die beabsichtigten Zwecke geeignet sind, haben sie noch erhebliche Nachteile. So kommt es z. B. bei den vorbekannten Vorrichtungen nicht immer zu einer vollständigen Entleerung der abgemessenen Flüssigkeitsmenge aus der Meßkammer. Auch arbeiten sie auf die Dauer in vielen Fällen nicht mehr meßgenau. Dies trifft insbesondere auf Konstruktionen mit porösen Elementen zu, deren Speicherfähigkeit und Durchlässigkeit sich im Laufe der Zeit verändert. Zudem sind die bekannten Konstruktionen noch ziemlich kompliziert, wodurch der wirtschaftlichen Herstell-und Montierbarkeit relativ enge Grenzen gesetzt sind.

Es besteht deshalb insbesondere die Aufgabe, eine Vorrichtung der eingangs erwähnten Art zu schaffen, welche die vorbeschriebenen Nachteile vermeidet und insbesondere eine möglichst vollständige Entleerung der abgemessenen Flüssigkeitsmenge aus der Meßkammer sowie eine dauerhaft hohe Meßgenauigkeit gewährleistet, wobei diese Vorrichtung gleichzeitig unkompliziert und wirtschaftlich herstell-und montierbar sein soll.

Diese Aufgabe wird erfindungsgemäß insbesondere dadurch gelost, daß die Meßkammer von einem rohrartigen äußeren Wandbereich und mindestens einem sich dahinein erstrekkenden Vorsprung in radialer Erstreckung begrenzt ist, wobei eine mit dem Inneren des Behälters verbundene Öffnung und eine die Spendekammer von der Meßkammer abteilende Ventilsitzplatte vorgesehen sind, und daß ein flexibler Befeuchtungsdeckel auf der dem Behälter abgewandten Seite der Vorrichtung oberhalb der Ventilsitzplatte angeordnet ist, wobei dieser Befeuchtungsdeckel ein Ventilelement zum Öffnen und Verschließen einer Ventilöffnung der Ventilplatte aufweist. Dadurch, daß diese Vorrichtung ein Kontroll-Ventilelement enthält, welches normalerweise die Spende-und die Meßkammer gegeneinader abdichtet, wird ein exakter Meßprozeß ermöglicht und mit Hilfe der weiteren Merkmale, insbesondere dem flexiblen Befeuchtungsdeckel wird eine praktisch vollständige Entleerung der abgemessenen Flüssigkeitsmenge erreicht. Eine vorteilhafte Weiterbildung der Vorrichtung wird durch Merkmale des zweiten Anspruches in Verbindung mit denen des ersten Anspruches erreicht. Insbesondere begünstigt die trichterförmige Ausgestaltung des die Meß-und Spendekammer trennenden Teils eine vollständige Entleerung der abgemessenen Flüssigkeitsmenge aus der Meßkammer in die Spendekammer bei entsprechender Betätigung des Deckelventilelementes. Die Konstruktion ermöglicht es auch, auf poröse Teilelemente ,insbesondere auf einen porösen Deckel, wie in US-Patent Nr. 4 133 614 dargestellt, zu verzichten.

Zweckmäßigerweise kann der Befeuchtungsdeckel aus elastischem Material, wie z. B. Gummi bestehen, welches entsprechende Lochungen aufweist. Es wird keine Flüssigkeit im Befeuchtungsdeckel selbst aufgenommen oder gespeichert, sondern die gesamte vorgemessene Menge kann durch die vorerwähnten Löcher im Gummi vollständig abgegeben werden.

Da abgemessene Flüssigkeitsmengen innerhalb der Vorrichtung durch einzelne Teileelemente nicht aufgesogen oder gespeichert werden, wird die Möglichkeit geschaffen, die abgemessene Dosis auch visuell zu bestimmen. Es ist deshalb zweckmäßig, wenn zumindest der äußere Randbereich der Meßkammer aus zumindest durchscheinendem Material besteht. Das einfache Konstruktionsprinzip ermöglicht eine umkomplizierte und wirtschaftliche Herstellung und Montage.

Weitere Ausgestaltungen der Erfindung sind in den Unteransprüchen ausgeführt.

Nachstehend ist die Erfindung in den ihr als wesentlich zugehörigen Einzelheiten anhand von erfindungsgemäßen Ausführungsbeispielen und der Zeichnungen noch näher beschrieben.

Es zeigen:

Fig. 1 eine in einem Teilbereich geschnittene Seitenansicht einer erfindungsgemäßen Meß-und Spendeapparatur,

Fig. 2 eine Draufsicht auf die Apparatur nach Fig. 1,

Fig. 3 eine perspektivische Explosionsdarstellung, mit Meßkammer und Spendekopf,

Fig. 4 eine vorgrößerte Teilansicht entlang der Linien 4 -4 von Fig. 1,

Fig. 5 eine vergrößerte Teilansicht ähnlich wie Fig. 4, welche das Deckelventil in offener Stellung zeigt,

Fig. 6 einen Teilquerschnitt einer modifizierten Ausführung einer erfindungsgemäßen Meß-und Spendeapparatur, die ein Deckelventil in geschlossener Position zeigt und

Fig. 7 eine Teilansicht ähnlich der von Fig. 6, die das Deckelventil in offener Spendestellung zeigt.

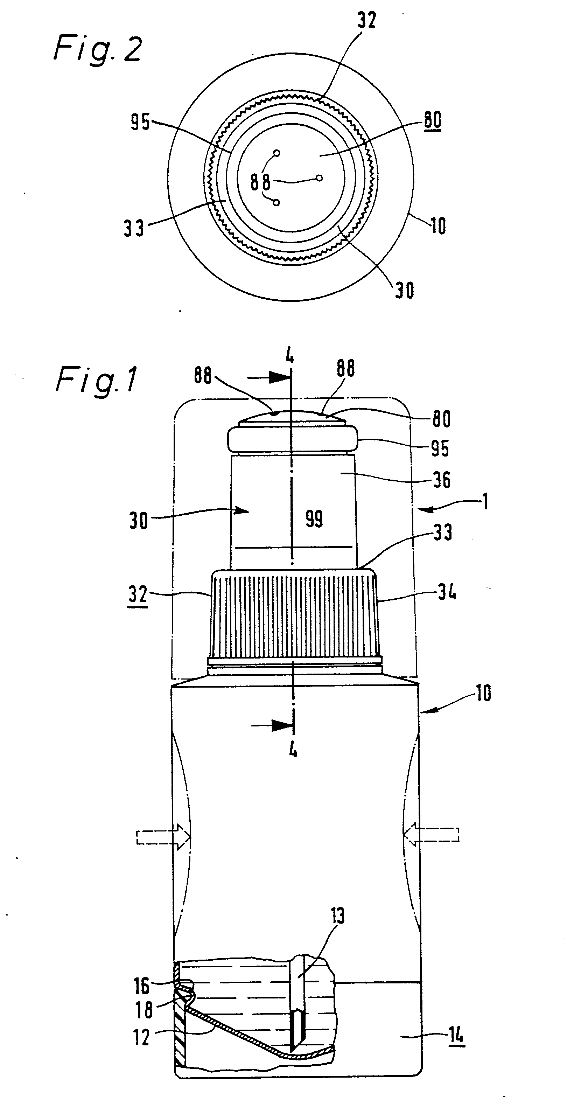

In den Fig. 1 bis 5 ist eine im ganzen mit 30 bezeichnete Meß-und Spendevorrichtung zum Gebrauch auf kompressiblen Behältern für Flüssigprodukte dargestellt. Es handelt sich bevorzugt um ein Ausführungsbeispiel zum Spenden von solchen Flüssigkeiten wie zum Beispiel von Medikamenten, die zur äußerlichen Anwendung auf menschliche Haut vorgesehen sind. Der Behälteraufsatz der vorliegenden Erfindung ist besonders für den Gebrauch von Medikamenten geeignet, bei denen es erwünscht und/oder notwendig ist, genau kontrollierte, abgemessene Mengen abzugeben, insbesondere bei periodischer Anwendung dieser genauen Mengen über eine ausgedehnte Zeitperiode hinweg. Der flexible Behälter 10 ist vorzugsweise aus Kunststoff hergestellt, wie z. B. Polyäthylen,und besitzt im allgemeinen einen runden Querschnitt sowie im vorliegenden Beispiel einen konischen Sockel 12, um die Entnahme des gesamten Behälterinhaltes durch eine Tauchröhre in später im einzelnen beschriebener Art und Weise zu erleichtern. Ein Anpaßstück in der Form eines Aufsatzes 14, der mittels einer Sockelrippe 16 und einer Sockelausnehmung 18 am konischen Sockel 12 verrastet ist, dient als Ständer, um die Packung in einer aufrechten Stellung zu halten. Der Behälter 10 hat (vgl z. B. Fig.4 u.5) einen aufrechtstehenden Halsbereich 20, der eine Entnahmeöffnung 22 bildet. Der Hals 20 ist mit einem üblichen äußeren Drehgewinde 24 versehen, so daß er durch eine ein inneres Drehgewinde aufweisende übliche Kappe oder dergl. verschlossen werden kann.

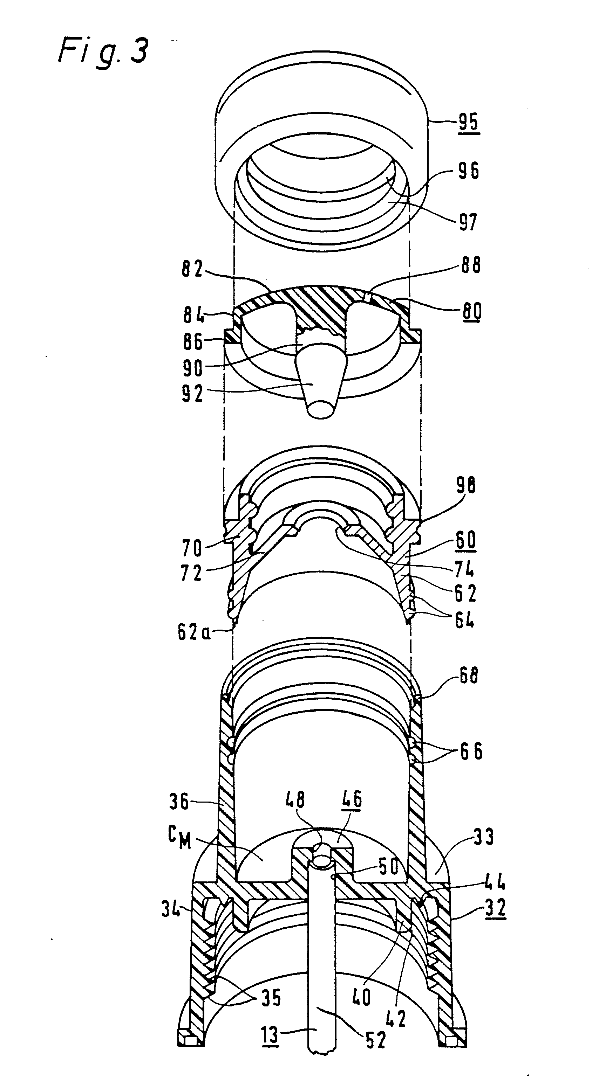

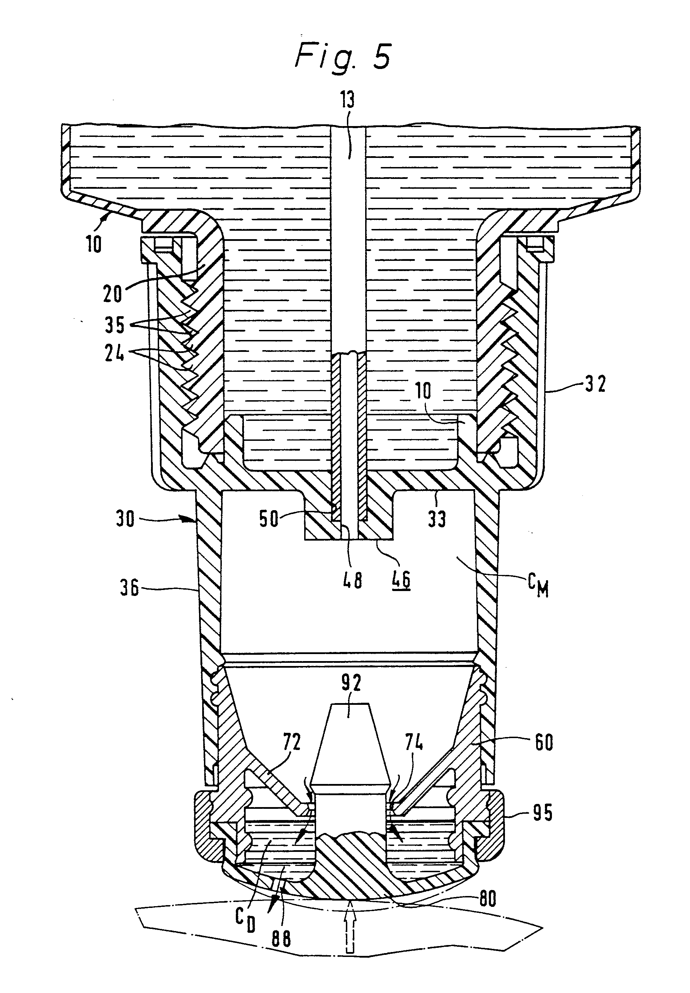

Erfindungsgemäß ist die Meß-und Spendevorrichtung 30 durch neue Konstruktions-und Anordnungsmerkmale gekennzeichnet, die für eine genaue Abgabe von vorgewählten und vorgemessenen Mengen von flüssigen Produkten sorgen. Die Anordnung ist auch ziemlich einfach konstruiert, so daß sie leicht und wirtschaftlich herzustellen und auf den Behälter der oben beschriebenen Art aufzusetzen ist. Die im ganzen mit 30 bezeichnete Vorrichtung ist aufgrund hygienischer Gesichtspunkte sowie wegen der erforderlichen Meßgenauigkeit durch ein neuartiges Dichtungssystem gekennzeichnet. Diesbezüglich enthält die Anordnung eine Kombinationskappen-, Meß-und Spendekammer-Unteranordnung, die,wie am besten in Fig. 3 dargestellt, einen Kappenbereich 32 in Form einer umgekehrten Schale umfaßt, der aus einer Deckenwand 33 und einem hiermit verbundenen unteren Sockel 34 mit einem inneren Gewinde 35 besteht, das in ein äußeres Gewinde 24 des Behälters 10 eingreift, um auf diese Weise für eine einfache Montage der Vorrichtung 30 über der Behälterentnahmeöffnung 22 zu sorgen. Die Vorrichtung 30 umfaßt weiterhin eine senkrechte,in der Regel zylinderische Wand 36, die sich aufwärts gerichtet von der äußeren Oberfläche der Deckenwand 33 des unteren Kappenbereiches 32 abhebt. Der Kappenbereich 32 enthält einen im wesentlichen zylindrischen mit ihm verbundenen Flansch 40, der eine abgewinkelte bzw. abgeschrägte untere Endkante 42 aufweist, und der innerhalb des Behälterhalses 20 anliegt und zusammen mit der radial auswärts gerichteten auf der axialen Stirnseite des Behälterhalses 20 aufliegenden Dichtrippe 44 für einen flüssigkeitsdichten Abschluß zwischen der Meß-und Spendevorrichtung 30 und dem Behälter 10 sorgt, sobald die Kappe 32 so wie in den Fig. 4, 5 dargestellt, auf dem Behälterhals 20 fest aufgeschraubt ist.

Wie in den Fig. 3 -5 dargestellt, hat der Kappenbereich 32 einen behälterachsenparallel aufwärts gerichteten Meßvorsprung 46, der zentral in der Meßkammer (CM) angeordnet ist und eine axial orientierte Auslaßöffnung 48 aufweist. Die Auslaßöffnung 48 hat, wie dargestellt, einen vergrößerten Innenbereich 50, um eine Röhre 52 aufzunehmen, welche dazu dient, den flüssigen Inhalt aus dem Behälter 10 in die Meßkammer CM zu saugen. Die Höhe des Meßvorsprunges 46 relativ zur Ebene der äußeren Oberfläche der Deckenwand 33 bestimmt zusammen mit dem inneren Durchmesser der Meßkammer CM das kontrollierte, vorbemessene Flüssigkeitsvolumen, das bei einmaliger Abmessung maximal gespendet werden kann. Natürlich kann dieses Volumen in einer Kombinationskappen-und Spendekammerunteranordnung durch einfache Veränderung der Höhe des Meßvorsprunges 46 und/oder des Durchmessers der Meßkammer CM variiert werden.

Im vorliegenden Beispiel enthält die Vorrichtung 30 weiterhin ein im wesentlichen verlängert rohrförmig ausgebildetes Ventilsitzplattenteil 60, das einen unteren abgewinkelten Plattenbereich 62 aufweist, sowie im vorliegenden Beispiel ein Paar axial angordneter, kreisförmig radial nach außen vorstehender Dichtrippen 64, die in die innere Ausnehmung 66 der inneren Wand der Meßkammer CM eingreifen. Zu beachten ist, daß die zylindrische Wand 36, die die Meßkammer CM umschließt, sich gegen ihre obere offene Endkante hin verjüngt und am Punkt 68 abgestuft ist, um einen vergrößerten Führungsbereich zu bilden, welcher das Einsetzen und die Montage der Ventilsitzplatte 60 erleichtert. Die Ventilsitzplatte 60 hat,wie dargestellt, eine abgestufte Schulter 70, die nahe an ihrem oberen Ende sitzt und außen um die äußere Seitenwand der Spendekammer CD verläuft (vergl. Fig. 4, 5). Die Ventilsitzplatte 60 weist eine innere abgestufte kegelstumpfförmige Wand 72 auf, die abgeschrägte Wandbereiche 72a, 72b mit jeweils unterschiedlicher Neigung und eine zentrale den Ventilsitz bildende Öffnung 74 hat. Zu beachten ist, daß die abgeschrägten Wandbereiche 72a, 72b eine praktisch vollständige Entleerung der Meßkammer gewährleisten. Die Ventilsitzplatte 60, die in ihrem oberen Bereich die äußere Wand 63 der Spendekammer CD bildet, ist durch einen Befeuchtungsdeckel 80 mit einer kreisförmigen Seitenwand 81 überdeckt, der aus nachgiebigem Material, z.B. Gummi, hergestellt ist und einen Kuppenbereich 82 aufweist, an den sich ein zylindrischer Wandbereich 84 anschließt, der in eine radial auswärts gerichtete, kreisförmig sich erstreckende Deckellippe 86 übergeht. Der Kuppenbereich 82 hat Deckelöffnungen 88 zur Flüssigkeitsabgabe, die im vorliegenden Beispiel in Form von Reihen kreisförmig angeordnet sind. Die Deckelöffnungen 88 sind vorzugsweise senkrecht zur außen konvex geformten Oberfläche 82a gerichtet. Durch diese Formgebung wird das Ventil 92 in Normalstellung geschlossen gehalten bzw. nach Anwendung, wie sie in Fig. 5 dargestellt ist, wieder in eine geschlossene Stellung zurückgebracht. Zentral zu den Entleerungsöffnungen weist der Kuppenbereich 82 einen nach innen in Richtung des Behälters 10 sich anschließenden Ventilschaft 90 mit einem vergrößerten, spitz zulaufenden,kegelstumpfförmigen Ventilbereich 92 auf,der sich auf der behälterseitigen Seite der Ventilsitzplatte 60 und der Ventilsitzöffnung 74 befindet und in Normalstellung zur Einnahme einer Dicht-oder Sitzposition vorgespannt ist (Fig. 4). Der Deckel ist abnehmbar durch einen Haltering 95 befestigt, der einen kurzen, radial einwärts gerichteten äußeren Ringflansch 96 aufweist, der gegen die -auf einer Abstützfläche 87 aufliegenden-Deckellippe 86 des Befeuchtungsdeckels 80 ein Widerlager bildet. Zusätzlich ist der Befeuchtungsdeckel 80 mittels eines abgeschrägten unteren Rastvorsprunges 97 gesichert,der mit einer kreisförmig sich erstreckenden Rastausnehmung 98 in der äußeren umschließenden Schulter 70 der Ventilsitzplatte 60 in Wirkverbindung steht, um die beschriebenen Teile in ihrer jeweiligen Stellung gegeneinander zu fixieren (vgl. Fig. 4 u. 5).

Die die erfindungsgemäße Meß-und Spendevorrichtung umfassenden Elemente können leicht und schnell auf einem Behälter montiert und angebracht werden. Die Ventilsitzplatte 60 wird z. B. dadurch auf den unteren Kappenbereich 32 montiert, daß sie soweit in ihn hineingeschoben wird, bis die Dichtrippen 64 einschnappen und in den Ausnehmungen 66 der Innenseite der zylinderischen Wand 36 der Meßkammer CM sitzen. In dieser Stellung sitzt die Dichtwulst 62a am unteren Ende des Plattenbe reiches 62 auf einer kreisförmig sich erstreckenden Stützrippe 36a auf der Innenseite der zylindrischen Wand 36 der Meßkammer CM auf und dichtet sie nach außen ab. Der Befeuchtungsdeckel 80 wird demnach einfach dadurch auf der Ventilsitzplatte 60 montiert, daß er über dem offenen Ende der Ventilsitzplatte 60 in Stellung gebracht wird, worauf der Befeuchtungsdeckel 80 und der Haltering 95 an den jeweils zugehörigen Platz gedrückt werden. Sodann wird die Tauchröhre 13 in den vergrößerten Innenbereich 50 des Meßvorsprunges 46 steckt. Die Anordnung kann dann auf einen kompressiblen Behälter 10 aufgebracht werden und die gesamte Einheit ist daraufhin fertig zum Gebrauch. Im folgenden sei kurz das Verfahren zum Gebrauch einer solchermaßen aus der erfindungsgemäßen Vorrichtung und einem Behälter bestehenden Packung betrachtet. Ist die im ganzen mit 1 bezeichnete Packung in einer aufrechten Stellung, so wie in Fig. 1 dargestellt; so drückt der Benutzer einfach auf die Seitenwand des nachgiebigen Behälters 10, so daß sich eine Flüssigkeitsströmung durch die Röhre 13 in die Meßkammer CM ergibt, bis sich darin eine abzumessende Flüssigkeitsmenge, wie in Fig. 4 gezeigt, angesammelt hat. Sobald die Kompressionsbewegung aufhört, fließt jeglicher Flüssigkeitsüberschuß durch die Röhre 13 zurück in den Behälter, so daß die in der Meßkammer CM verbleibende Menge genau der benötigten Menge entspricht. Auf der Wand 36 der Meßkammer CM kann ein Hinweis in Form einer Linienmarkierung 99 vorgesehen sein, um dem Benutzer eine visuelle Hilfe dafür zu geben, daß auch die für eine gegebene Anwendung gewünschte exakte Menge aus dem Behälter entnommen worden ist (vergl. Fig.1). Die Anordnung wird sodann auf den Kopf gestellt, womit die Flüssigkeit zur Decke der durch das Ventil 92 des Befechtungsdeckels 80 ab gedichteten Meßkammer fließt. Wird der Befeuchtungsdeckel 80 nun gegen die Haut des Benutzers gedrückt, so wird der Ventilschaft 90 einwärts versetzt, so daß die abgemessene Flüssigkeitsmenge in die Spendekammer CD hinter den Befeuchtungsdeckel fließen kann. Der Befeuchtungsdeckel 80 wird dann einfach über die Hautoberfläche bewegt, bis die zuvor abgemessene Menge durch die Deckelöffnungen 88 ausgetreten ist.

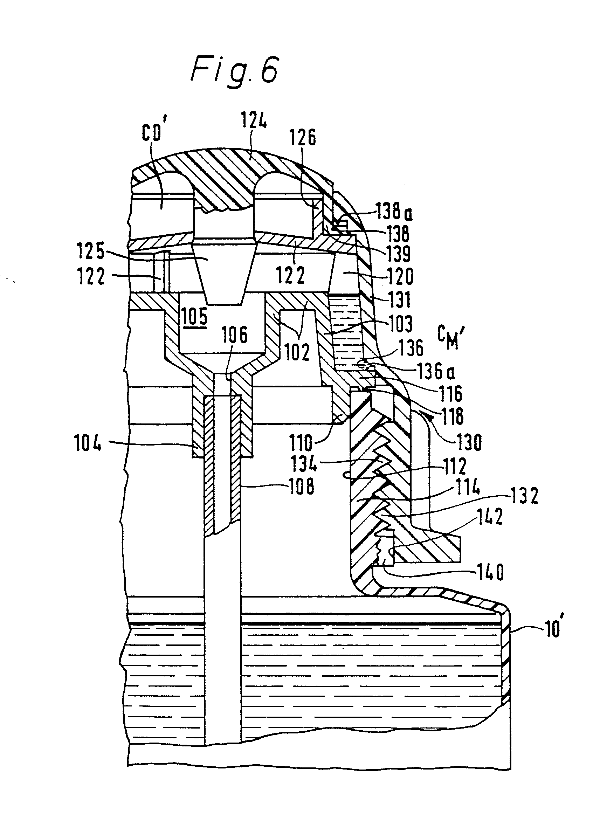

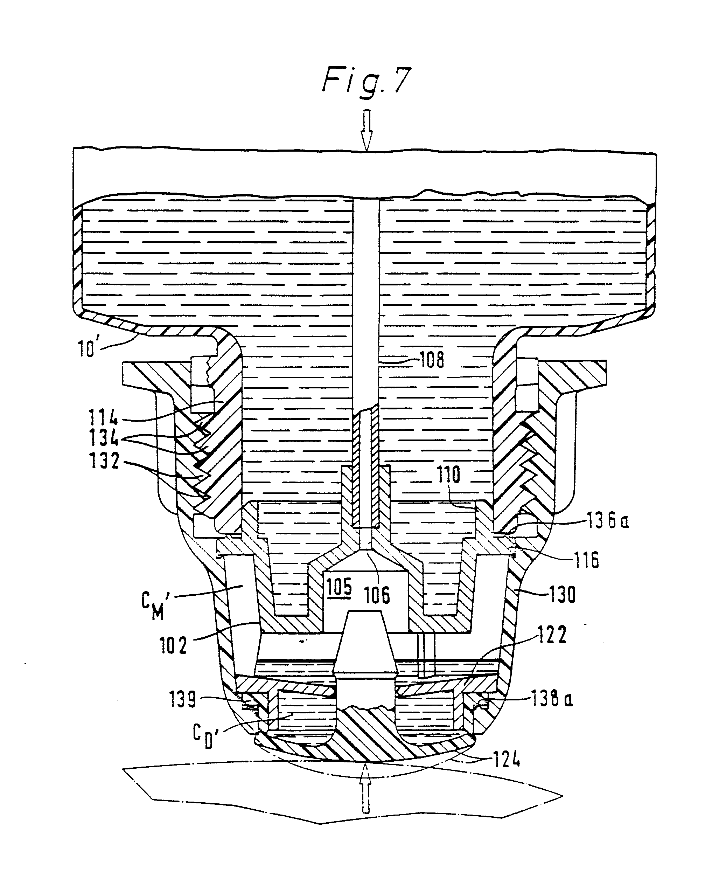

In den Fig. 6 und 7 ist eine andere Ausgestaltung einer erfindungsgemäßen Meß-und Spendeanordnung zur Montage auf eine Mündung eines kompressiblen Behälters dargestellt. Der Behälter 10' ist mit dem vorstehend Beschriebenen identisch. Die Meß-und Spendevorrichtung 30' ist jedoch in ihren Komponenten und ihrer Anordnung in diesem Ausführungsbeispiel etwas verschieden. Im vorliegenden Beispiel enthält die Vorrichtung 30' eine Meßwanne 102 mit einem im wesentlichen M-förmigen Querschnitt, die einen sich zentral in Richtung zum Behälter anschließenden Kragen 104 aufweist. Die Meßwanne 102 ist in ihrem Mittelstück als zum Ventil 125 konzentrisch ausgerichtete Ventilausnehmung 105 ausgeformt, die das Ventil beim Eindrücken des als Ventildeckel 124 ausgebildeten Befeuchtungsdeckels aufnehmen kann. Hierdurch wird eine kompakte Anordnung dieser Elemente erreicht. Die Ventilausnehmung 105 hat in Richtung zum Kragen 104 einen sich verjüngenden Sockel, der in einer, eine Flüssigkeitsaustrittsöffnung bildenden Zentralöffnung 106 endet. Die Zentralöffnung 106 ist in Richtung zum Behälter hin vergrößert, um eine Tauchröhre 108 aufzunehmen, welche sich zum Behälterboden erstreckt, um, falls gewünscht, die Flüssigkeit von dort zu entnehmen. Die Meßwanne 102 hat einen sich an sie anschließenden und kreisförmig sich in die Behältermündung hinein erstreckenden Dichtflansch 110, der an der Behälterhalts-Innenseite 112 anliegt, sowie einen radial auswärts gerichteten Seitenflansch 116 mit einer auf seiner unteren Oberfläche befindlichen Unterwulst 118, die der axialen Stirnfläche der Behältermündung gegenüberliegt und diese beaufschlagt.

Im vorliegenden Ausführungsbeispiel übergreift der Ventildeckel 124 eine kreisförmig aufrechtstehende Ventilsitzplattenwand 126 der Ventilsitzplatte 122 und wird durch eine Überkappe 130 mit innerem Gewinde 132, welches mit einem äußeren Gewinde 134 auf der Behälteroberfläche zusammenwirkt, fest und dicht gegen die Ventilsitzplatte 122 gedrückt. An der Überkappe 130 sind zur axialen Abstützung der Ventilsitzplatte 122 drei kreisförmig angeordnete, nach innen gerichtete, axial verlaufende Stege 120 angebracht. Die Überkappe 130 hat, wie in Fig. 6 u.7 dargestellt, zwei Dichtschultern 136, 138 mit kreisförmig sich erstreckenden Schulterwülsten 136a, 138a, die die Ventilsitzplatte 122 bzw. die Oberseite des Seitenflansches 116 beaufschlagen und dadurch den durch die Meßwanne 102 und die Ventilsitzplatte 122 umgrenzten Raum an den Verbindungsflächen zur Überkappe 130 sicher abdichten. Der Behälterhals hat radial gegenüberliegende, radial vorstehende in axiale Ausnehmungen 142 passenden Zähne 140, die im unteren Teil der Überkappe 130 ausgeformt sind, um die Montage zu ermöglichen und daraufhin zur Verschlußsicherung der Teile zu dienen. Hierdurch wird die Vorrichtung kindersicher. In der hier vorliegenden erfindungsgemäßen Ausgestaltung ist die Meßkammer CM' in radialer Erstreckung zwischen einer innenliegenden äußeren Wand 103 der Meßwanne 102 und einem Wandabschnitt 131 der Überkappe 130 angeordnet. Um eine vorgesehene abzumessende Menge der Flüssigkeit abzugeben, braucht der Benutzer lediglich einen einwärts gerichteten Druck auf die Seitenwände des Behälters 10' auszuüben, wodurch die Flüssigkeit aufwärts durch die Zentralöffnung 106 in die Meßkammer CM fließt. Wenn die Kammer CM bis zur Randebene der Meßschüssel 102 gefüllt ist, wird jede weitere Flüsigkeit durch die zentral vergrößerte Ventilausnehmung 105 in den Behälter zurückfließen. Nun dreht der Benutzer einfach die Vorrichtung 30' um und drückt das Ventil gegen die Stelle der Hautoberfläche, an der ein Auftrag der Flüssigkeit gewünscht ist, worauf sich das Ventil öffnet und die abgemessene Menge in die Sammel-oder Spendekammer CD hinter den Ventildeckel 124 und sodann auswärts durch die daran befindlichen Öffnungen auf die Haut des Benutzers fließt.

Alle in den Ansprüchen und in der Beschreibung genannten Merkmale der Erfindung können einzeln und im Zusammenhang erfindungswesentlich sein.

**Claims**

1. Vorrichtung (30) zum Dosieren und Spenden von Flüssigkeiten, z. B. von kosmetischen Emulsionen oder medizinischen Lösungen auf die Haut eines Anwenders, mit einem unteren Kappenbereich (32) und Befestigungselementen (35) zu lösbaren Befestigen auf der Entnahmeöffnung eines Behälters (10), wobei in einem oberen Kappenbereich eine Meßkammer (CM) zum Abmessen einer vorgesehenen Flüssigkeitsmenge angeordnet ist, sowie eine Spendekammer (CD) mit einem durchlässigen Befeuchtungsdeckel (80), **dadurch gekennzeichnet,** daß die Meßkammer (CM) von einem rohrartigen äußeren Wandbereich (36) und mindestens einem sich dahinein erstreckenden Vorsprung (46) in radialer Erstreckung begrenzt ist, wobei eine mit dem inneren des Behälters (10) verbundene Öffnung (38) und eine die Spendekammer (CD) von der Meßkammer (CM) abteilende Ventilsitzplatte (60) auf der dem Behälter (10) abgewandten Seite der Vorrichtung (30) oberhalb der Ventilsitzplatte (60) angeordnet ist, wobei dieser Befeuchtungsdeckel ein Ventilelement (92) zu Öffnen und Verschließen einer Ventilöffnung (74) der Ventilplatte (60) aufweist.

2. Vorrichtung nach Anspruch 1, dadurch gekennzeichnet, daß der Wandbereich (36) der Meßkammer (CM) vorzugsweise etwa behälter-achsparallel angeordnet ist und sich mindestens ein Vorsprung (46) zweckmäßigerweise in azialer Richtung erstreckt und daß die die lichte Öffnung des äußeren Wandbereiches (36) auf der dem Behälter (10) abgewandten Seite überdeckende Ventilsitzplatte (60) einen in behälterabgewandter Richtung konusförmig zusammenlaufenden Platten bereich - (42) aufweist, der in der Ventilöffnung (74) endet, wobei diese (74) und ihr zugehöriges Ventilelement (92) so aufeinander abgestimmt sind, daß bei einwärts gerichteter Betätigung des Befeuchtungsdeckels (80) ein Durchlaß von der Maßkammer - (CM) in die durch die Innenwand des Befeuchtungsdeckels (80) und die Wände der Ventilsitzplatte - (60) gebildete Spendekammer (CD) freigegeben wird.

3. Vorrichtung nach Anspruch 1 oder 2, dadurch gekennzeichnet, daß der untere Kappenbereich (32) eine obere, die Behältermündung überdeckende Deckenwand (33) mit einem kreisförmig und senkrecht von der Decke sich erstreckenden Flansch (40) aufweist, der innen an der Entnahmeöffnung des Behälters (10) dichtend anliegt.

4. Vorrichtung nach einem der Ansprüche 1 bis 3, dadurch gekennzeichnet, daß sich eine Dichtrippe (44) konzentrisch aussen um den Flansch - (40) erstreckt und die stirnseitige Oberfläche der Behältermündung dichtend beaufschlagt.

5. Vorrichtung nach einem der Ansprüche 1 bis 4, dadurch gekennzeichnet, daß die Ventilsitzplatte (60) auf ihrem äußeren Umfang herausstehende, in axialer Erstreckung versetzt angeordnete Dichtrippen (64) aufweist, die dichtend und stabilisierend in dazu passende innere Ausnehmungen (66) im Inneren der zylinrischen Wand (36) einrastbar sind.

6. Vorrichtung nach einem der Ansprüche 1 bis 5, dadurch gekennzeichnet, daß die der Behältermündung zugewandte untere Endkante der Ventilsitzplatte (60) eine kreisförmig sich erstreckende Dichtwulst (62a) aufweist, die im Inneren der kreisförmigen Wand (36) eine Stützrippe (36a) beaufschlagt.

7. Vorrichtung nach einem der Ansprüche 1 bis 6, dadurch gekennzeichnet, daß die Ventilsitzplatte (60) eine zur Behälterachse parallele kreisförmige Spendekammerwand (63) aufweist, die sich aufwärts in behälterabgewandte Richtung hin erstreckt und zur Abstützung der Seitenwand (81) des Befeuchterdeckels (80) dient.

8. Vorrichtung nach einem der Ansprüche 1 bis 7, dadurch gekennzeichnet, daß der Befeuchterdeckel (80) eine kreisförmige, radial auswärtsgerichtete Deckellippe (86) aufweist, die auf einer in dem Ventilsitzelement (60) eingeformten Abstützfläche (87) aufliegt und von dem radial einwärtsgerichteten Ringflansch (96) eines die Deckellippe (86) umschließenden Halteringes (95) übergriffen ist, wobei vorzugsweise der Haltering - (95) einen Rastvorsprung (97) aufweist, der in eine Ausnehmung (98) der Ventilsitzplatte (60) einrastbar ist.

9. Vorrichtung nach Anspruch 1 oder einem der Ansprüche 2 bis 8, dadurch gekennzeichnet,daß sie eine Meßwanne(102) mit einem im wesentlichen M-förmigen Querschnitt aufweist, die axial zwischen dem Behälter (10) und einer Ventilsitzplatte (122) angeordnet ist und worin die Meßkammer (CM') in radialer Erstreckung zwischen einer innenliegenden äußeren Wand (103) der Meßwanne (102) und einem Wandabschnitt (131) einer Überkappe (130) angeordnet ist, die radial aussen um die Meßwannenwand (103) herum verläuft und die Ventilsitzplatte (122) über der Entnahmeöffnung des Behälters (10) hält.

10. Vorrichtung nach Anspruch 9, dadurch gekennzeichnet, daß die Überkappe (130) eine abgestufte Konfiguration mit axial beabstandeten, einwärtsgerichteten Schultern (136, 138) aufweist, die kreisförmig sich erstreckende Schulterwülste (136a, 138a) aufweisen, wobei eine Schulterwulst - (136a) die äußere Kante der Meßschüssel (102) beaufschlagt und sie gegen die Stirnfläche der Behältermündung drückt, während die andere Dichtwulst (138a) eine radial um den Ventildeckel - (124) herum verlaufende Deckellippe (86') beaufschlagt und sie gegen die Ventilsitzplatte ( 122) drückt.

11. Vorrichtung nach Anspruch 9 oder 10, dadurch gekennzeichnet, daß sie Halteelemente in der Form von Stegen (120) aufweist, die oberhalb des obersten Flüssigkeits-Meßpegels etwa radial einwärtsgerichtet aus der Überkappe (130) hervorstehen und als Widerlager zur Abstützung der Ventilsitzplatte (122) dienen.

12. Vorrichtung nach einem der Ansprüche 9 bis 11, dadurch gekennzeichnet, daß das vorzugsweise mit dem Ventildekkel (124) verbundene Ventil (125) in eine durch einen zentralen taschenartigen Mittelbereich der im Querschnitt M-förmigen Meßschüssel (102) gebildete Ventilausnehmung ( 105) eingreift.

Fig. 2

Fig. 1

*Fig. 3*

Fig. 4

## Fig. 5

# Fig.6

Fig.7